# EUROPEAN PATENT APPLICATION

(11) **EP 2 682 112 A1**
(43) Date of publication of application: **08.01.2014**
(21) Application number: 13175057.2
(22) Date of filing: 04.07.2013
(51) Int. Cl.: A61K 9/48, A61K 31/192, A61K 31/426, A61K 9/16, A61K 9/20, A61K 9/28

(54) **Combined capsule formulations of flurbiprofen and famotidine**

(30) Priority: 05.07.2012 TR 201207840
(71) Applicant: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Mutlu, Onur, 34460 Istanbul (TR)
(74) Representative: Sevinç, Erkan

(57) **Abstract**

The present invention relates to a capsule formulation comprising, together with an NSAID with anti-inflammatory, analgesic and antipyretic activities, an H2 receptor antagonist used for preventing or minimizing gastrointestinal side effects caused by NSAID.

## Description

### Technical Field:

The present invention relates to a capsule formulation comprising, together with an NSAID with anti-inflammatory, analgesic and antipyretic activities, an H2 receptor antagonist used for preventing or minimizing gastrointestinal side effects caused by NSAID.

### Background of the Invention

Flurbiprofen (Formula 1) is a propionic acid derivative, also known as NSAID (non-steroidal anti-inflammatory drug), with the analgesic, anti-inflammatory and antipyretic activities.

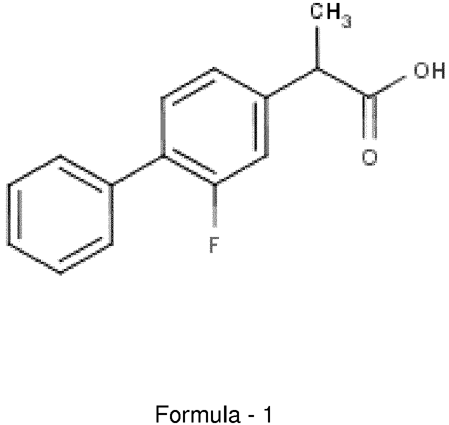

Flurbiprofen is used to alleviate pain in musculoskeletal system and joint disorders such as ankylosing spondylitis, osteoarthritis and rheumatoid arthritis; in soft tissue injuries such as sprains and strains; and for postoperative pain, as well as pains including migraine headache and for painful and difficult menstruation. At the same time, flurbiprofen is used as a pastille for the symptomatic healing of sore throat. Besides tablet and pastille forms, gel, capsule and solution forms are also among the dosage forms of flurbiprofen.

Besides the wide area of usage, flurbiprofen also has a wide range of side effects. These side effects are mostly seen in the gastrointestinal system and the most frequent one effects the stomach mucosa. These side effects can be observed as gastric erosion, peptic ulcer, major upper gastrointestinal system bleedings, alterations in the intestine permeability and inflammation. These side effects are caused by the fact that flurbiprofen inhibits cyclooxygenase enzyme activity and prostaglandin synthesis, thereby damaging gastrointestinal mucosa due to increased gastric acid level. In fact, the mechanism of effect of flurbiprofen in the treatment is realized by the inhibition of prostaglandins and cyclooxygenase enzymes present in the tissues. However, when the same enzymes also present in the gastrointestinal mucosa cells are inhibited, the side effects listed above are observed.

In order to prevent or minimize the side effects emerged as a result of the increase in the acid level caused by NSAID molecules such as flurbiprofen, a lot of studies have been carried out in prior art. For example, in order to minimize gastric damage, flurbiprofen is enteric coated (Hashmat D., Shoaib H., Mehmood Z. (2008) AAPS PharmaSci Tech 9(1): 116-121) or formulated such that controlled release thereof will be performed (EP0234670). Moreover, the researchers have combined NSAID molecules with acid inhibitors, in addition to formulation studies performed, in order to prevent or treat gastrointestinal damage. Acid level of gastric fluid is balanced, with acid inhibitors in this combination. Patents numbered US5204118 and US5417980 belonging to the state of the art combine NSAID molecules with proton pump inhibitors, so as to balance gastric acid. Since the researches on this combination have been carried out for many years, NSAID molecules have been combined with many molecules balancing gastric acid so far. The most important ones of these molecules may be listed as proton pump inhibitors, H2 receptor antagonists, and prostaglandin analogues.

In order to effectively administer the studied NSAID - acid inhibitor combinations to the human body, various studies for multilayer tablet formulations have also been carried out. One of the primary reasons for this case is to lessen or prevent incompatibility of the substances to be formulated with one another. Moreover, another different reason is that the formulations of two active substances with different release properties can be provided in the same form. The patent numbered EP1411900 relates to a multilayer tablet comprising NSAID and acid inhibitors. Here, NSAIDs are present in the inner core and released after acid inhibitors in the outer layer. Furthermore, as an alternative to multilayer tablet formulations, pharmaceutical formulations comprising enteric coated proton pump inhibitor and NSAID have been developed, in order to prevent gastrointestinal side effects that are likely to be caused by NSAID (US2003129235). Since proton pump inhibitors are adversely affected by gastric acid, these molecules should be released at higher pH. For this purpose, enteric coated formulations of these molecules have been developed.

Despite all these developments, in order to minimize or prevent gastrointestinal side effects that are likely to be caused by NSAIDs, a need for novel pharmaceutical formulations combined with acid inhibitors, as well as for novel stable pharmaceutical formulations in which these combined molecules are released at the desired time and potential incompatibilities are prevented, still exists today.

### Detailed Description of the Invention

The present invention relates to a pharmaceutical formulation comprising, together with an NSAID with anti-inflammatory, analgesic and antipyretic activities, an H2 receptor antagonist used for preventing or minimizing gastrointestinal side effects caused by NSAID. Here, H2 receptor antagonist decreases acid level of gastric fluid by inhibiting acid secretion, thereby preventing or minimizing such diseases as gastric erosion, peptic ulcer, major upper gastrointestinal system bleedings, alterations in the intestine permeability and inflammation, all of which are caused by NSAID.

According to another aspect, the present invention relates to a pharmaceutical formulation in which NSAID is formulated in a way to provide sustained release, in order to prevent or minimize gastrointestinal side effects caused by it. NSAID at a therapeutic rate is preserved by means of sustained release. In addition, side effects and their severity increasing with instant release of NSAID in gastrointestinal system are minimized.

According to another aspect, the present invention relates to the fact that the pharmaceutical formulation comprising NSAID as first active substance and H2 receptor antagonist as second active substance is a novel oral dosage form. This oral dosage form may be tablet, capsule, pellet, granule, tablet in tablet, tablet in capsule, or coated tablet, preferably being in capsule form.

The present invention relates to oral capsule formulation comprising NSAID with anti-inflammatory, analgesic, and antipyretic activities; wherein NSAIDs are selected from the group including aspirin, acetaminophen, ibuprofen, flurbiprofen, ketoprofen, naproxen, oxaprozin, etodolac, indomethacin, ketolorac, lornoxicam, nabumetone, or diclofenac; the said NSAID being preferably flurbiprofen.

The present invention relates to an oral capsule formulation comprising a H2 receptor antagonist increasing gastric acid level; wherein H2 receptor antagonists are selected from cimetidine, ranitidine, ebrotidine, pabutidine, lafutidine, loxtidine, or famotidine; the said H2 receptor antagonist being preferably famotidine.

The present invention may comprise flurbiprofen and famotidine molecules in the pharmaceutical formulation as pharmaceutically acceptable salts, enantiomers, racemates, polymorphs, esters, and/or hydrates thereof.

According to another aspect, the present invention relates to oral capsule formulation comprising i) sustained release pellet comprising flurbiprofen as first active agent and pharmaceutically acceptable excipient and ii) tablet comprising famotidine as second active agent and pharmaceutically acceptable excipient. Oral capsule formulation according to the invention has been developed such that it will comprise flurbiprofen and famotidine molecules in the same dosage form, yet separately. Thus, potential incompatibility of flurbiprofen and famotidine molecules, used in combination to minimize or prevent gastrointestinal side effects that are likely to be caused by flurbiprofen, is eliminated and at the same time, they are optionally enabled to have different release properties.

According to the invention, pharmaceutically acceptable excipient can be selected from the group including, but not limited to, binding agents, dispersants, glidants, lubricants, plasticizers, surface active agents, preservatives, controlled release agents, adsorbents, or mixtures thereof.

Suitable binders, may include, but not limited to, polymetacrylate, polyvinylpyrrolidone (povidon), hydroxypropyl methyl cellulose (HPMC), hydroxypropyl cellulose (HPC), carboxy methyl cellulose (CMC), methyl cellulose (MC), hydroxy ethyl cellulose, sodium carboxy methyl cellulose (NaCMC), carboxymethyl cellulose calcium, ethyl cellulose, polyethylene oxide, gelatin, starch, pregelatinized starch, xanthan gum, guar gum, alginate, carrageenan, pectin, carbomer, cellulose acetat phytalate, hydroxy propyl starch, polaxomer, poly ethylene glycol or mixtures thereof.

Suitable disintegrants of the invention, may include, but not limited to, microcrystalline cellulose, croscarmellose sodium, xylitol, polyplasdone (1-ethenylpyrrolidin-2-one), crospovidone, low-substituted hydroxypropyl cellulose (L-HPC) and sodium starch glycolate or mixtures thereof.

Suitable glidants of the invention may include, but not limited to, silicon dioxide, magnesium trisilicate, starch, talc, colloidal silicon dioxide or silicon hydrogel or mixtures thereof.

Suitable lubricants of the invention, may include, but not limited to, magnesium stearate, sodium stearyl fumarate, polyethylene glycol, stearic acid, metal stearates, boric acid, sodium chloride benzoate and acetate, sodium or magnesium lauryl sulfate or mixtures thereof.

Suitable plasticizers of the invention may comprise, but not limited to, triethyl citrate, triacetin, citric acid esters, phthalic acid esters dibutyl sebacate, cetyl alcohol, polyethylene glycol, polysorbate or mixtures thereof.

Suitable surface active agents of the invention, may include, but not limited to, dioctyl sulfosuccinate, polysorbates and polyoxyethylene alkyl esters and ethers thereof, glyceryl monolaurate saponins, sorbitan laurate, sodium lauryl sulfate, magnesium lauryl sulfate or mixtures thereof.

Suitable preservatives of the invention, may include, but not limited to, methyl paraben, propyl paraben and salts thereof (e.g. sodium or potassium salts), sodium benzoate, citric acid, benzoic acid, butylated hydroxytoluene and butylated hydroxyanisole or mixtures thereof.

Suitable controlled release agents of the invention may include, but not limited to, cellulose acetate phthalate, ammonium methacrylate copolymer, cellulose acetate trimellitate, hydroxypropyl methylcellulose phthalate, polyvinyl acetate phthalate, hydroxypropyl methylcellulose acetate succinate, hydrogels, carboxyvinyl polymer, sodium alginate, sodium carmellose, calcium carmellose, polyvinyl alcohol, gelatine, starch, polyvinylpyrrolidone, microcrystalline cellulose, collagen, hydroxypropyl methylcellulose, hydroxyethyl cellulose, hydroxypropyl cellulose, carboxymethyl cellulose, carnauba wax, cocoa oil, paraffin, hydrogenated vegetable oil, cetyl alcohol, stearyl alcohol, stearic acid, xanthane gum or mixtures thereof.

Suitable adsorbents of the invention may include, but not limited to, silicon dioxide, kaoline, magnesium aluminum silicate, cyclodextrin, alginic acid, propylene glycol alginate, gum, microcrystalline cellulose, ethyl cellulose, methyl cellulose, calcium carboxymethyl cellulose, hydroxypropyl methyl cellulose, poloxamer, hydroxymethyl cellulose, hydroxypropyl cellulose, povidone or mixtures thereof.

The oral capsule according to the invention comprises pellets which contain, as active substance, flurbiprofen and pharmaceutically acceptable excipient which are formulated so as to provide sustained release of flurbiprofen. Flurbiprofen sustained release pellets according to the present invention may be formulated in a way to have i) a neutral core that does not comprise active substance and ii) a layer comprising active substance, plasticizer, controlled release agent, adsorbent, and optionally, some other pharmaceutically acceptable excipient.

Flurbiprofen sustained release pellets according to the present invention comprises a neutral core surrounded by the active layer comprising active substance. Here "active layer" indicates comprising active substance while "neutral core" indicates not comprising active substance.

Neutral core according to the invention consists of microcrystalline cellulose as pharmaceutically acceptable excipient, forming approximately 15-35% of the pellet formulation by weight.

The present invention relates to oral capsule comprising flurbiprofen sustained release pellets comprising neutral core with an average particle size of 400-800 µm, and preferably of 500-750 µm and an active layer comprising flurbiprofen coating these pellets. Release profile of flurbiprofen released from pellets obtained as a result of coating the neutral cores which have these particle values, being coated by an active layer, increases with the increasing surface area.

Flurbiprofen sustained release pellets according to the invention comprise an active layer surrounding neutral core wherein the layer provides controlled release agent as pharmaceutically acceptable excipient. Controlled release agent comprised by flurbiprofen sustained release pellets of the present invention is methacrylate copolymer, and more preferably ammonium methacrylate copolymer. Here, methacrylate copolymers include ethyl acrylate, methyl methacrylate, methacrylic acid ester and/or mixtures thereof.

Flurbiprofen sustained release pellets according to invention comprise at least two methacrylate copolymers in active layer; these methacrylate copolymers are selected from a mixture including poly(ethyl acrylate-co-methyl methacrylate -co-trimethylaminoethyl methacrylate chloride)(1:2:0,2) and poly(ethyl acrylate-co-methyl methacrylate-co-trimethylaminoethyl methacrylate chloride)(1:2:0,1) copolymers. At least two methacrylate copolymers according to the invention are at least 1:1 by weight.

Flurbiprofen sustained release pellets according to the invention comprises, together with methacrylate copolymers as controlled release agent, triethyl citrate as plasticizer; wherein the said methacrylate copolymers are between approximately 20 and 70 mg by weight, and triethyl citrate is between approximately 5 and 20 mg by weight.

Flurbiprofen sustained release pellets according to the invention are formulated in a way to minimize or prevent gastrointestinal side effects that are likely to be caused by flurbiprofen; wherein methacrylate copolymer as controlled release agent and triethyl citrate as plasticizer have a value varying between 0,5 to 7, when they are proportioned by weight. Methacrylate copolymer and triethyl citrate having this rate form a matrix formulation where therapeutic rate of flurbiprofen molecule is released sustainably; and apart from designing release profile, it is provided that flurbiprofen molecule inside the matrix is stabilized.

Flurbiprofen sustained release pellets according to the invention are coated with silicon dioxide, preferably with silicon dioxide solution of 10%, in order to increase stability.

Famotidine tablet according to the invention comprises an active core and a neutral coating that surrounds this core. Here "active core" indicates that the core comprises active substance while "neutral coating" indicates that no active substance is present in the coating.

The coating in the famotidine tablet according to the invention comprises, as pharmaceutically acceptable excipient, hydroxypropyl methylcellulose, titanium dioxide, polyethylene glycol and/or mixture thereof. By means of this coating, the interaction of famotidine molecule with flurbiprofen is prevented, and at the same time famotidine is enabled to remain stable in the tablet.

According to another aspect, the present invention relates to an oral capsule formulation of flurbiprofen and famotidine, developed for minimizing gastrointestinal side effects that are likely to be caused by flurbiprofen; characterized in that flurbiprofen sustained release pellets comprise methacrylate copolymer and triethyl citrate in active layer while famotidine tablet comprises hydroxypropyl methyl cellulose, titanium dioxide and polyethylene glycol in neutral coating. Thus, flurbiprofen and famotidine molecules are formulated in a way not to interact, and at the same time it is provided that these molecules remain stable in their own form.

Outer layer of flurbiprofen sustained release pellets according to the invention surrounding the neutral core comprises;
i) flurbiprofen approximately 50-400 mg by weight as active substance,
ii) triethyl citrate approximately 5-20 mg by weight as plasticizer,
iii) methacrylate copolymers approximately 20-70 mg by weight as controlled release agent,
iv) silicon dioxide approximately 0.5-5 mg by weight as adsorbent.

Famotidine tablet formulation according to the invention comprises;
i) famotidine approximately 20-80 mg by weight as active substance,
ii) pregelatinized starch approximately 10-50 mg by weight as binding agent,
iii) microcrystalline cellulose approximately 10-50 mg by weight as dispersant,
iv) talc approximately 0.5-5 mg by weight as glidant,
v) magnesium stearate approximately 0.1-5 mg by weight as lubricant.

According to another aspect, the present invention is a method for preparing oral capsule, comprising the following steps:
a) for sustained release pellets comprising flurbiprofen;
   i. triethyl citrate and water are mixed,
   ii. flurbiprofen is added in portions onto the mixture obtained in step i),
   iii. methacrylate copolymers are added to the mixture obtained in step ii) and mixed,
   iv. neutral pellets comprising microcrystalline cellulose are prepared,
   v. pellets are coated by spraying the mixture obtained in step iii) onto the neutral pellets,
   vi. silicon dioxide is added onto the pellets obtained in step v).
b) for tablet comprising famotidine;
   i. famotidine, pregelatinized starch and microcrystalline cellulose are mixed,
   ii. the mixture obtained in step i) is granulated with water,
   iii. granules obtained in step ii) are dried and sieved,
   iv. talc and magnesium stearate are added to the granules obtained in step iii) and they are compressed and made into tablet,
   v. the tablet obtained in step iv) is coated with a cellulose based coating.
c) capsule filling is performed with the pellets obtained in step a) and tablet obtained in step b).

### Examples

In the present invention, to enhance the stability, to prevent the interaction between active ingredients and to provide different release properties of them, the following examples are defined. Although the examples are not intended to limit the scope of the present invention, it should be considered in the light of the description detailed above.

### Example 1

The oral capsule formulation of the present invention comprises a film coated tablet of famotidine and pellets of flurbiprofen. The film coated tablet of famotidine is provided in Table 1, and pellets of flurbiprofen in Table 2. The preparation of the famotidine tablet and flurbiprofen pellets is detailed in the description.

**Table 1 - Famotidine film coated tablet**

| **Ingredients** | **Amount (mg)** |
|---|---|
| **Core** | |
| Famotidine | 40.00 |
| Pregelatinized starch | 32.00 |
| Microcrystalline cellulose | 23.20 |
| Talc | 1.20 |
| Magnesium stearate | 0.60 |

| **Cellulose Based Film Coating** | |
|---|---|
| Hydroxypropyl methylcellulose | 1.86 |
| Titanium dioxide | 0.87 |
| Polyethylene glycol | 0.24 |
| Yellow dye | 0.03 |

**Table 2 - Flurbiprofen pellets**

| **Ingredients** | **Amount (mg)** |
|---|---|
| Flurbiprofen | 200.00 |
| Microcrystalline cellulose | 68.00 |
| Triethyl citrate | 11.54 |
| Ammonium methacrylate copolymer mixture [Eudragit RL30D:Eudragit RS30D (1:1)] | 45.88 |
| Silicon dioxide | 1.58 |

### Example 2

The non-interaction of flurbiprofen and famotidine is determined by the following dissolution tests which are carried out by the USP2 paddle dissolution method. Flurbiprofen dissolution from the capsule of the present invention is compared with the reference drug which comprises only flurbiprofen pellets (Table 3). It is shown that flurbiprofen of the present invention has a similar dissolution profile with reference drug and that famotidine does not affect flurbiprofen dissolution in the present invention. In the same manner, famotidine dissolution profile comparison given in Table 4 indicates the non-interaction between flurbiprofen and famotidine.

**Table 3 - Dissolution profile of flurbiprofen suitable for the present invention compared to reference drug. (pH=7.2, 0.05 M KH2PO4 buffer, 900 mL, pallet, 50 rpm)**

| | **% Dissolution of Flurbiprofen** | |
|---|---|---|
| **Time** | **Reference - Flurbiprofen 200 mg** | **Flurbiprofen 200 mg-Famotidine 40 mg Capsule** |
| 0 | 0,0 | 0,0 |
| 15 | 4,1 | 5,8 |
| 30 | 7,1 | 11,2 |
| 45 | 10,9 | 16,2 |
| 60 | 14,8 | 21,2 |
| 90 | 21,4 | 28,8 |
| 120 | 28,1 | 35,8 |
| 180 | 38,8 | 46,1 |
| 240 | 48,8 | 55,2 |
| 300 | 57,9 | 62,8 |
| 360 | 66,5 | 69,2 |
| 420 | 73,7 | 75,0 |
| 480 | 79,4 | 79,6 |
| 720 | 92,5 | 90,4 |

**Table 4 - Dissolution profile of famotidine suitable for the present invention compared to reference drug. (pH:1.2, 900 mL, 50 rpm)**

| | **% Dissolution of Famotidine** | |
|---|---|---|
| **Time** | **Reference - Famotidine 40 mg** | **Flurbiprofen 200 mg-Famotidine 40 mg Capsule** |
| 0 | 0 | 0 |
| 5 | 93 | 87 |
| 10 | 97 | 97 |
| 15 | 99 | 101 |
| 20 | 99 | 101 |
| 30 | 100 | 102 |
| 45 | 101 | 103 |

### Example 3

The following Table 5 and Table 6 is about dissolution profile of flurbiprofen and famotidine depending on time. These tests are carried out by USP2 Pallet Dissolution Method and show that quality of the composition is provided and that the capsule formulation of the present invention and its release profile are stable.

**Table 5 - Stability tests at 25ºC - %60 RH, 30ºC - %65 RH, 40ºC - %75 RH**

| **Conditions** | **Time** | **%Dissolution of flurbiprofen in 0 month** | **%Dissolution of flurbiprofen in 3 months** | **%Dissolution of flurbiprofen in 6 months** |
|---|---|---|---|---|
| 25ºC, %60 RH (relative humidity) | 60 minutes | %18 | %18 | %17 |
| | 240 minutes | %41 | %50 | %47 |
| | 960 minutes | %86 | %81 | %90 |
| 30ºC, %65 RH | 60 minutes | %18 | %20 | %18 |
| | 240 minutes | %41 | %53 | %48 |
| | 960 minutes | %86 | %80 | %90 |
| 40ºC, %75 RH | 60 minutes | %18 | %18 | %18 |
| | 240 minutes | %41 | %50 | %49 |
| | 960 minutes | %86 | %79 | %90 |

**Table 6 - Stability tests at 25ºC - %60 RH, 30ºC - %65 RH, 40ºC - %75 RH**

| **Conditions** | **Time** | **%Dissolution of famotidine in 0 month** | **%Dissolution of famotidine in 3 months** | **%Dissolution of famotidine in 6 months** |
|---|---|---|---|---|
| 25ºC, %60 RH (relative humidity) | 45 minutes | %81 | %82 | %102 |
| 30ºC, %65 RH | 45 minutes | %81 | %85 | %103 |
| 40ºC, %75 RH | 45 minutes | %81 | %83 | %104 |

## Claims

1. An oral capsule comprises,
i) sustained release pellet comprising, flurbiprofen as an active substance and pharmaceutically acceptable excipient/s, and
ii) tablet comprising, famotidine as an active substance and pharmaceutically acceptable excipient/s.

2. An oral capsule according to claim 1; wherein the pellet comprises a neutral core and an active layer surrounding the neutral core.

3. An oral capsule according to claim 2; wherein the neutral core comprises microcrystalline cellulose as excipient.

4. An oral capsule according to claim 2; wherein the average particle size of the neutral core is 400-800 µm, and preferably 500-750 µm.

5. An oral capsule according to claim 2; wherein the active layer comprises at least two methacrylate copolymers as excipient.

6. An oral capsule according to claim 5; wherein at least two methacrylate copolymers are selected from a mixture including poly(ethyl acrylate-co-methyl methacrylate -co-trimethylaminoethyl methacrylate chloride)(1:2:0,2) and poly(ethyl acrylate-co-methyl methacrylate-co-trimethylaminoethyl methacrylate chloride)(1:2:0,1) copolymers.

7. An oral capsule according to claims 5 and 6; wherein the rate of the two methacrylate copolymers is 1:1 by weight.

8. An oral capsule according to claims 2 to 7; wherein the active layer comprises at least two methacrylate copolymers and triethyl citrate as excipient.

9. An oral capsule according to claim 8; wherein the rate of the mixture of at least two methacrylate copolymers to triethyl citrate is 0.5 to 7 by weight.

10. An oral capsule according to claim 1; wherein the tablet comprises an active core and a neutral coating that surrounds this core and said neutral coating does not have an active substance.

11. An oral capsule according to claim 10; wherein the coating comprises hydroxypropyl methyl cellulose, titanium dioxide, polyethylene glycol and/or mixtures thereof.

12. An oral capsule according to any one of the preceding claims; **characterized in** having;
i) sustained release pellets comprising an active layer that comprises flurbiprofen as active substance, and methacrylate copolymer and triethyl citrate as pharmaceutically acceptable excipient, and a neutral core surrounded by this coating, and
ii) tablet comprising an active core comprising famotidine as active substance and a neutral coating that surrounds the core and that comprises hydroxypropyl methyl cellulose, titanium dioxide and polyethylene glycol as pharmaceutically acceptable excipient.

13. An oral capsule according to any one of the preceding claims; **characterized in** having;
i) sustained release pellets comprising an active layer that comprises
a. flurbiprofen as an active substance,
b. methacrylate copolymer,
c. triethyl citrate and
d. silicon dioxide
and a neutral core comprising microcrystalline cellulose, and
ii) tablet comprising an active core comprising
a. famotidine as active substance,
b. pregelatinized starch,
c. microcrystalline cellulose,
d. talc, and
e. magnesium stearate,
and neutral coating that surrounds the active core and that comprises,
a. hydroxypropyl methyl cellulose,
b. titanium dioxide, and
c. polyethylene glycol.
